# EUROPÄISCHE PATENTSCHRIFT

(11) **EP 0 808 179 B1**
(45) Veröffentlichungstag und Bekanntmachung des Hinweises auf die Patenterteilung: **10.01.2001**
(21) Anmeldenummer: 96904007.0
(22) Anmeldetag: 07.02.1996
(51) Int. Cl.: A61L 11/00, C02F 1/02

(54) **VORRICHTUNG UND VERFAHREN ZUM STERILISIEREN VON ABWASSER**
DEVICE AND PROCESS FOR THE STERILIZATION OF WASTE WATER
PROCEDE ET DISPOSITIF POUR LA STERILISATION D'EAUX RESIDUAIRES

(30) Priorität: 10.02.1995 DE 19504479; 26.06.1995 DE 29510060 U
(43) Veröffentlichungstag der Anmeldung: 26.11.1997
(73) Patentinhaber: IC Ingenieur Consult Technische Gesamtplanung GmbH, 60433 Frankfurt (DE)
(72) Erfinder: SCHMIDT, Reinhard, D-61352 Bad Homburg (DE)
(74) Vertreter: Stoffregen, Hans-Herbert, Dr. Dipl.-Phys. Patentanwalt
(86) Internationale Anmeldenummer: EP9600502
(87) Internationale Veröffentlichungsnummer: WO9624390

(56) Entgegenhaltungen:
- EP-A- 0 020 157
- DE-A- 2 503 605

## Beschreibung

Die Erfindung bezieht sich auf eine Vorrichtung zum Sterilisieren von Abwasser wie infektiösem oder gentechnisch belastetem Abwasser umfassend zumindest einen ersten und einen zweiten Aufnahmebehälter, sowie auf ein entsprechendes Verfahren.

Insbesondere im Bereich der Forschung und Entwicklung können sich Anforderungen in Laboratorien im Zuge neuer Forschungsprojekte ändern. Hierdurch bedingt kann sich mit der Umstellung bzw. Veränderung der Arbeitsgebiete auch die Gefahrenklasse ändern. Dies macht dann, wenn zum Beispiel hochgradig infektiöse oder gentechnisch belastete Abwasser entsorgt werden sollen, umfassende Umbaumaßnahmen im Entwässerungssystem erforderlich. Hierzu werden stationär Sterilisationsvorrichtungen eingebaut, mittels derer das zu entsorgende Abwasser sterilisiert wird. Bei einer Vielzahl von Laborplätzen erfordert dies aufwendige bauliche Änderungen, auch dann, wenn an Laborplätzen nur zeitweise entsprechende Abwässer anfallen sollten.

Aus der DE 41 26 619 A1 ist eine Sterilisiervorrichtung zum Sterisilieren von Flüssigkeit durch kontinuierliche Erwärmung bekannt, bei der die zu sterilisierende Flüssigkeit einer Dampfstrahlpumpe zugeführt wird, die ausgangsseitig eine Heizvorrichtung aufweist.

In der DE 25 41 601 A1 wird eine Wasseraufbereitungsanlage mit einem Boiler sowie Vorratsbehälter beschrieben, die gegebenenfalls mobil ausgebildet sein kann.

Um Wasser zu sterilisieren, ist nach DE-PS 60 232 ein verfahrbarer Wagen vorgesehen, in dem mehrere Behälter in Reihe geschaltet sind, durch die das zu sterilisierende Wasser strömt. Mittels zweier in Reihe geschalteter Dampfkessel kann Flüssigkeit auf über 120 °C erwärmt werden, um dieses zu sterilisieren (DE-PS 58 829).

Eine weitere Vorrichtung zum Sterilisieren von Wasser ist der DE-PS 146 402, DE-PS 62 114 oder der Literaturstelle GWF, Wasser, Abwasser, 134 (1993), Nr. 1, Seiten 1 bis 9 zu entnehmen.

Der vorliegenden Erfindung liegt das Problem zugrunde, eine Vorrichtung der eingangs genannten Art so weiterzubilden, daß auf einfache, jedoch sichere Weise Abwässer, insbesondere infektiöse oder gentechnisch hochbelastete Abwässer sterilisiert und entsorgt werden können, ohne daß hierzu bauliche Veränderungen am zu entsorgenden Arbeits- wie Laborplatz erforderlich sind. Auch soll eine Vereinfachung der Sterilisation von Abwässern dann erfolgen, wenn an verschiedenen Orten nur zeitweise Abwässer anfallen.

Erfindungsgemäß wird das Problem dadurch gelöst, daß jeder Aufnahmebehälter der verfahrbar ausgebildeten Vorrichtung als Abwasser sammelnder kontrolliert kühlbarer Sterilisationsbehälter ausgebildet ist und jeweils sowohl eine Heizeinrichtung als auch ein Kühlelement einer Kühleinrichtung aufweist.

Die Vorrichtung ist insbesondere als Untertisch ausgebildet, der eine obere Abdeckung aufweist, die mit einer das Abwasser in einen der Aufnahmebehälter leitenden Zuführung wie Trichter oder Becken versehen ist. Von dem Untertisch ausgehend im Bereich der Zuführung kann ein Wasserzapfhahn angeordnet sein, der einerseits an eine stationäre Leitung anschließbar und andererseits vorzugsweise eine Näherungsautomatik aufweist.

In der Abdeckung des Untertisches können Luftaustrittsöffnungen einer Kältemaschine vorhanden sein, in deren geschlossenen Kältekreislauf die Kühlelemente wie Kühlschlangen schaltbar sind.

Durch die erfindungsgemäße Lehre können Abwässer wie infektiöse oder gentechnisch hochbelastete Abwässer über die erfindungsgemäße Doppel-Aufnahmebehälteranlage für wechselnden Betrieb auf Sterilisationstemperatur aufgeheizt und anschließend nach der Sterilisationszeit über eine entsprechende Kühlung auf eine Temperatur abgekühlt werden, um das Abwasser dem normalen Entwässerungssystem zuführen zu können. Die Rückkühlung muß dabei nicht notwendigerweise über eine Rückkühleigenversorgung wie die Kältemaschine, sondern kann durch Leitungswasser erfolgen, welches die Kühlschlangen in den jeweiligen Aufnahmebehältern durchströmt.

Um das Abwasser zum Sterilisieren im erforderlichen Umfang zu erhitzen, ist bodenseitig in jedem Aufnahmebehälter ein Heizrohr angeordnet, das mit einem Kleindampferzeuger verbunden ist. Dieses ist über Steuerventile mit den Rohren verbunden. Der Kleindampferzeuger besteht aus einem vorzugsweise zylinderförmigen Gehäuse, in dem ein Heizstab angeordnet ist und über den der erforderliche Dampf erzeugt wird. Durch die Anordnung des Dampferzeugers außerhalb der Aufnahmebehälter ergibt sich eine Wartungsfreundlichkeit. Auch ist eine entsprechende Heizmittelanordnung weniger störanfällig.

Durch die erfindungsgemäße Vorrichtung wird erkennbar der Anforderung von Sicherheitsstandards für den Betrieb von Arbeitsplätzen entsprochen, an denen belastete Abwässer anfallen.

Um im gewünschten Umfang einen der Aufnahmebehälter zu entleeren, kann von jedem Aufnahmebehälter eine zum Beispiel über ein Magnetventil absperrbare Ableitung ausgehen, die in eine gemeinsame Leitung übergehen, in der ein Förderaggregat wie Zahnradpumpe angeordnet ist, um auf diese Weise das sterilisierte und im erforderlichen Umfang abgekühlte Abwasser abpumpen zu können. Anschließend wird das Abwasser über eine Verbindung wie Schlauch dem Entwässerungssystem zugeführt.

Um jedoch auch bei Undichtheiten der Verschlußorgane wie Magnetventile sicherzustellen, daß Abwasser nicht unkontrolliert in das Entwässerungssystem gelangen kann, endet der Schlauch oberhalb der Aufnahmebehälter in dem Entwässerungssystem.

Der Schlauch selbst kann über eine vorzugsweise an einer Seite des Untertischs angeordnete Schnellschlußkupplung mit der das Förderaggregat aufweisenden Leitung verbunden werden.

Bei der Verwendung einer Kältemaschine wird die von dem Verflüssiger kommende Leitung aufgezweigt und geht in die Kühlschlangen der Aufnahmebehälter über. Bodenseitig werden die Kühlschlangen in einer gemeinsamen Leitung nach außen geführt, die im Verdichter der Kältemaschine mündet.

In den aus dem Aufnahmebehälter kommenden Leitungen des Kältekreislaufs sind dann Absperrventile wie Regelventile vorgesehen, um wahlweise eine der Kühlschlangen und damit den Aufnahmebehälter in dem Kältekreislauf zu integrieren. Die Regelventile können über Temperaturfühler angesteuert werden, die im Kopfbereich der Aufnahmebehälter angeordnet sind und die Temperatur des Abwassers bestimmen.

Ferner ist im Kopfbereich eines jeden Aufnahmebehälters eine Füllstandssonde angeordnet, um sicherzustellen, daß ein unzulässiges Auffüllen des Aufnahmebehälters unterbleibt bzw. um nach Erreichen eines bestimmten Füllstandes den entsprechenden Aufnahmebehälter abzusperren und den anderen zum Beschicken mit Abwasser freizugeben.

Jeder Aufnahmebehälter besteht vorzugsweise aus Edelstahl und ist als Druckbehälter ausgebildet.

Das Verfahren zum Sterilisieren von vorzugsweise hochgradig infektiösem und/oder gentechnisch belastetem Abwasser zeichnet sich dadurch aus, daß das Abwasser wahlweise einem von zwei in einem verfahrbaren Untertisch angeordneten Aufnahmebehältern zugeführt wird, daß das zu sterilisierende Abwasser auf eine Temperatur T1 von zumindest 121°C, vorzugsweise 134° C erhitzt wird, daß nach erfolgter Sterilisation das Abwasser auf eine Temperatur T2 von zumindest 85° C, vorzugsweise zumindest 80° C abgekühlt wird, und sodann einem Entwässerungssystem zugeführt wird, wobei während des Abkühlens der andere Auffangbehälter mit weiterem zu sterilisierendem Abwasser gefüllt und/oder in dem Behälter eingefülltes Abwasser sterilisiert wird.

Vorzugsweise erfolgt die Sterilisation über eine Zeitdauer von 20 Minuten. Weitere Einzelheiten, Vorteile und Merkmale der Erfindung ergeben sich nicht nur aus den Ansprüchen, den diesen zu entnehmenden Merkmalen - für sich und/oder in Kombination -, sondern auch aus dem der nachfolgenden Beschreibung von der Zeichnung zu entnehmendem bevorzugten Ausführungsbeispiel.

Es zeigen:
- Fig. 1: eine Prinzipdarstellung einer ersten Ausführungsform eines Untertisch-Sterilisators in Seitendarstellung,
- Fig. 2: den Untertisch-Sterilisator nach Fig. 1 in Vorderansicht,
- Fig. 3: eine zweite Ausführungsform eines Untertisch-Sterilisators,
- Fig. 4: der Untertisch-Sterilisator nach Fig. 3 in Vorderansicht und
- Fig. 5: eine alternative Ausführungsform zu dem den Fig. 1 - 4 zu entnehmenden Untertisch-Sterilisatoren.

In Fig. list rein prinzipiell eine als verfahrbarer Untertisch (10) ausgebildete Vorrichtung (12) zum Sterilisieren von Abwässern, insbesondere hochgradig infektiösen bzw. gentechnisch belasteten Abwässern dargestellt.

Der Untertisch (10), dessen Korpus aus Edelstahl oder schlagfestem Kunststoff bestehen kann, ist auf Rollen (14), (16), (18) abgestützt, um ein leichtes Verfahren und Abstellen an Orten zu ermöglichen, an denen zu entsorgendes Abwasser anfällt.

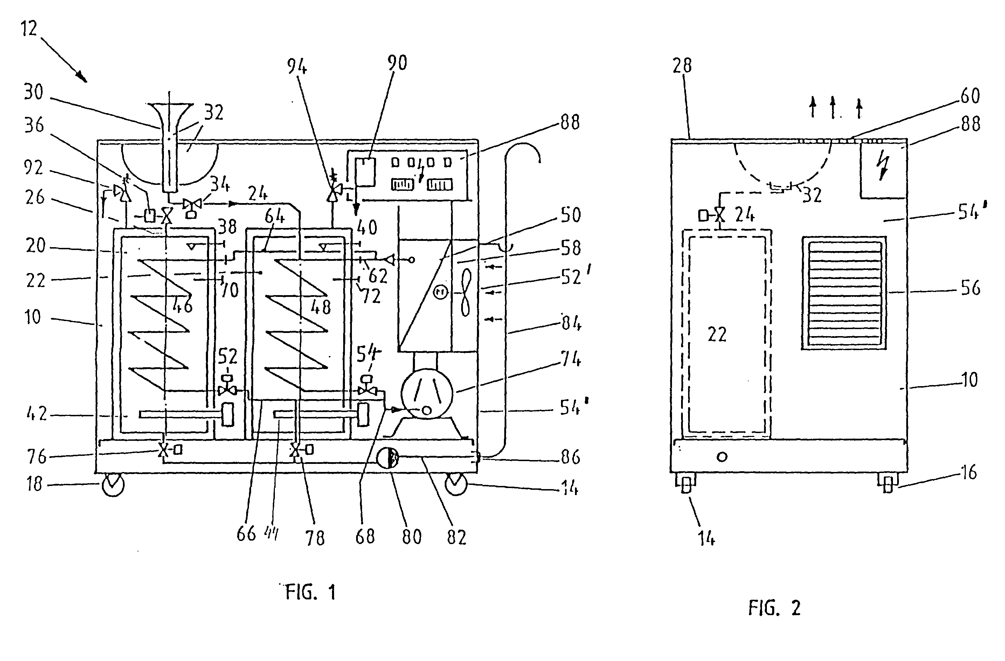

Die Vorrichtung (12) bzw. der Untertisch (10) weisen nach dem Ausführungsbeispiel der Fig. 1 und 2 als integrale Bestandteile zumindest zwei Aufnahme- bzw. Auffangbehälter (20), (22) auf, die als Druckbehälter ausgebildet sind und aus Edelstahl bestehen können. Die Aufnahmebehälter (20), (22) sind über Leitungen (24), (26) mit Abwasser befüllbar, welches zum Beispiel über einen Trichter (30) und/oder ein Edelstahlbecken (32) gesammelt und zugeführt wird. Dabei ist das Edelstahlbecken (32) bzw. der Trichter (30) in einer Abdeckung (28) des Untertischs (10) eingelassen bzw. an dieser befestigt.

Zwischen dem Zulauf des Abwassers und den Aufnahmebehältern (20) und (22) verlaufenden Leitungen (24) und (26) sind Magnetventile (34) und (36) angeordnet, um wahlweise einen der Aufnahmebehälter (20), (22) mit Abwasser zu befüllen oder aber in einem der Behälter die erforderliche Sterilisation vorzunehmen. In diesem Fall übt der andere Behälter die Funktion einer Auffangstation aus.

Selbstverständlich besteht auch die Möglichkeit des überlappenden Betriebs. Dies bedeutet, daß gleichzeitig oder zeitversetzt Abwasser in beiden Aufnahme- bzw. Auffangbehältern (20), (22) sterilisiert oder in einem der Behälter eine Sterilisation und in dem anderen eine Abkühlung sterilisierten Abwassers erfolgt.

Um ein Überfüllen der Aufnahmebehälter (20) bzw. (22) auszuschließen, sind Füllstandsanzeiger (38) und (40) vorhanden, die die Magnetventile (34), (36) steuern und gegebenenfalls parallel hierzu optische bzw. akustische Signale über den jeweiligen Füllstand abgeben,

Um das zu sterilisierende Abwasser im erforderlichen Umfang heizen zu können, befinden sich im Bodenbereich eines jeden Behälters (20), (22) Heizstäbe (42), (44) oder andere geeignete Heizelemente. Bei einem Volumen von 20 Litern eines jeden Aufnahmebehälters (20), (22) kann ein Heizstab (42), (44) einer Leistung von 4,5 kW eingesetzt werden, damit das Abwasser, also Entwässerungsgut zum Beispiel 20 Minuten auf eine Temperatur T1 = 121° C aufgeheizt und gehalten wird.

Grundsätzlich wird beim Sterilisationsbetrieb in einem der Behälter (20), (22) der andere Behälter (20), (22) entweder mit zu sterilisierendem Abwasser befüllt oder aber in diesem vorhandenes bereits sterilisiertes Abwasser rückgekühlt. Hierzu verlaufen in jedem Behälter (20), (22) Kühlschlangen (46), (48), die wahlweise in einen geschlossenen Kältekreislauf einer Kältemaschine (50) über Ventile (52), (54) zuschaltbar sind.

Die Kältemaschine (50), die im Ausführungsbeispiel luftgekühlt ist, weist einen üblichen Aufbau auf. Die erforderliche Kühlluft wird über einen Ventilator (52) zugeführt, der über in einer Seitenwandung (54') des Untenischs (10) vorhandene Lufteintrittsöffnung (56) Luft ansaugt. Die den Verflüssiger (58) der Kältemaschine (50) durchströmende Luft wird über eine Abluftöffnung (60) abgeleitet, die in der Abdeckung (28) des Untertisches (10) eingelassen ist.

Von dem Verflüssiger (58) wird Kältemittel über Leitungen (62), (64) wahlweise einer der Kühlschlangen (46) bzw. (48) zugeführt. Die Kühlschlangen (46), (48) gehen in Leitungen (66), (68) über, die über die Regelventile (52), (54) in Abhängigkeit von der über Temperaturfühler (70), (72) ermittelten Temperatur geöffnet bzw. abgesperrt werden. Vor der Kältemaschine (50), das heißt dessen Verdichter (74) werden die Leitungen (66), (68) zusammengeführt.

Das Rückkühlen des sterilisierten Abwassers erfolgt dabei in einem Umfang, daß das Abwasser auf eine Temperatur von in etwa 80°C abgekühlt wird. Sodann wird ein bodenseitig vorhandenes Magnetventil (76) bzw. (78) geöffnet, um das Abwasser mittels eines Förderaggregats wie Zahnradpumpe (80) über eine Leitung (82) abzusaugen und einem Schlauch zuzuführen, der in einem üblichen Entwässerungssystem mündet. Der Schlauch (84) kann dabei über eine an der Seitenwandung (54) vorhandene Schnellschlußkupplung (86) angeschlossen werden.

Der Schlauch (84) mündet in dem nicht dargestellten Entwässerungssystem oberhalb der Oberseiten der Auffangbehälter (20), (22), um sicherzustellen, daß bei möglicherweise vorhandenen Undichtigkeiten der Anschlüsse bzw. Ventile verunreinigtes Abwasser nicht unkontrolliert in das Entwässerungssystem fließen kann.

Ferner sind Steuerungs- sowie Regelelemente bzw. -organe im oberen Bereich des Untertisches (10) unmittelbar unterhalb der Abdeckung (28) angeordnet. Dies wird rein prinzipiell durch das Bezugszeichen (88) angedeutet.

Die Steuerung (88) umfaßt ferner einen Chargen-Schreiber (90), um Füllvorgänge, Zeit- und Temperaturvorgänge protokollieren zu können.

Schließlich ist jeder Sterilisationsbehälter (20), (22) mit einem Sicherheitsventil (92), (94) abgesichert.

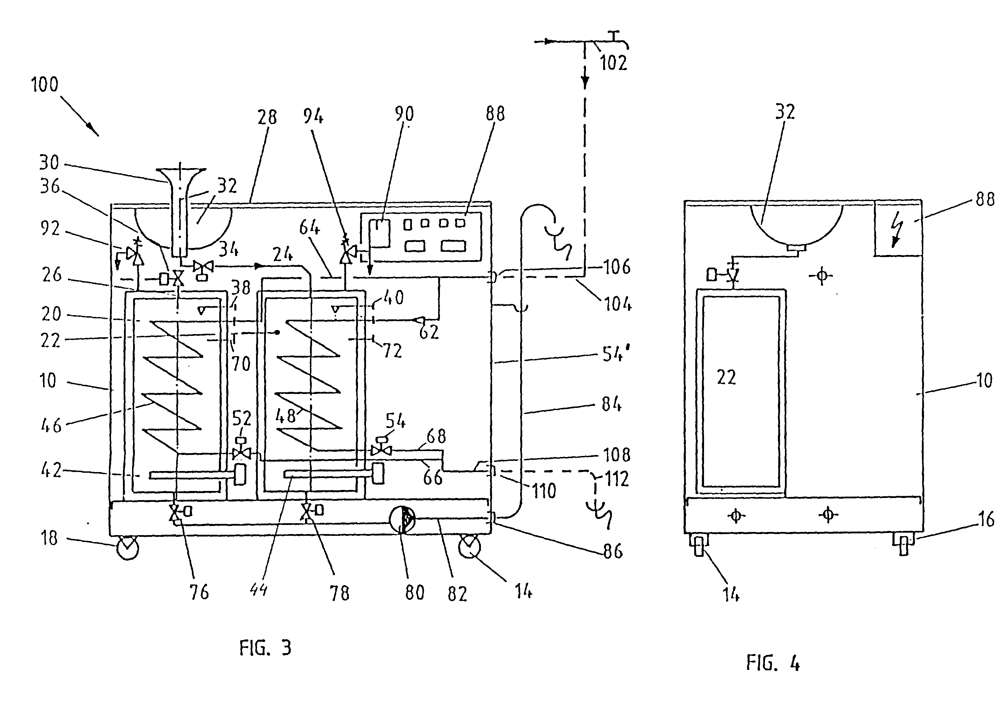

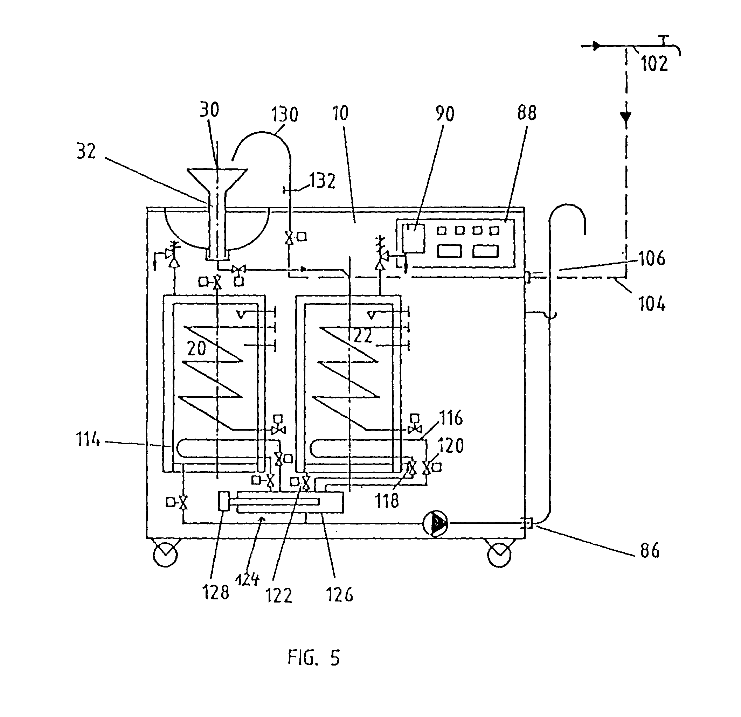

In den Fig. 3 und 4 ist eine zweite Ausführungsform einer Vorrichtung (100) dargestellt, die sich von der Vorrichtung (12) gemäß Fig. 1 und 2 nur dahingehend unterscheidet, daß zur Kühlung von in den Behältern (20) bzw. (22) vorhandenem Abwasser kein Kältemittel einer Kältemaschine, sondern Kühlwasser benutzt wird, welches zum Beispiel einem Trinkwassernetz (102) entnommen werden kann.

Das Kühlwasser wird über eine Leitung (104) geführt und über eine Schnellschlußkupplung (106) mit dem Untertisch (10) verbunden, um sodann in die Leitungen (62), (64) überzusehen, die in den Kühischlangen (46) und (48) münden. Entsprechend gehen die über die Ventile (52) und (54) absperrbaren Leitungen (66), (68) in eine Leitung (108) über, die über einen Anschlußstutzen (110) mit einem Schlauch (112) verbindbar ist, über den sterilisiertes und im erforderlichen Umfang abgekühltes Wasser dem normalen Entwässerungssystem zugeführt werden kann. Ansonsten ist die Funktion der Vorrichtung (100) entsprechend der Vorrichtung (12).

Fig. 5 ist insoweit eine Weiterbildung zu den Ausführungsformen der Fig. 1 und 2 zu entnehmen, als daß die Behälter (20), (22) nicht über Heizstäbe, sondern über im Bodenbereich verlaufende Heizrohre (114) und (116) erhitzt werden, die über Magnetventile (118), (120), (122) mit einem einzigen Kleindampferzeuger (124) verbunden sind. In Abhängigkeit von der Stellung der Steuerventile (118), (120), (122) wird einer der Behälter (20), (22) oder gegebenenfalls beide oder auch keiner mit vom Dampferzeuger (124) kommenden Dampf beaufschlagt.

Der Kleindampferzeuger (124) besteht aus einem zylindrischen Gehäuse (126), in dem vorhandenes vorzugsweise destilliertes Wasser über einen Heizstab (128) im erforderlichen Umfang verdampft wird.

In weiterer Ausgestaltung weist der Untertisch (10) einen Wasserzapfhahn (130) auf, der über die Leitung (104) mit einer stationären Wasserleitung (102) verbindbar ist. Der Wasserzapfhahn (130), der oberhalb des Trichters (30) bzw. Beckens (32) mündet, weist vorzugsweise eine Näherungsautomatik (132) auf, um Wasser fließen zu lassen bzw. dieses abzusperren.

Ansonsten entspricht die Ausführungsform der Fig. 5 denen der Fig. 1 - 4, so daß auf die weiteren Elemente nicht weiter einzugehen ist.

Typische Abmessungen des Untertisches (10) können sein:

| | |
|---|---|
| Baulänge | 900 mm |
| Bauhöhe | 850 mm |
| Bautiefe | 550 mm. |

Das Auffangvolumen eines jeden Behälters (20), (22), der aus Edelstahl besteht, kann 20 l betragen.

Zum Aufheizen des zu sterilisierenden Abwassers können die Heizstäbe (42), (44) eine Leistung von 4,5 kW aufweisen. Das Abwasser sollte über einen Zeitraum von 20 Minuten auf eine Temperatur von 121° C aufgeheizt und gehalten werden. Anschließend erfolgt ein Abkühlen des sterilisierten Abwassers auf eine Temperatur von etwa 80° C. Der gesamte Vorgang, das heißt das Aufheizen, das Halten des Abwassers auf der Temperatur von 121° C und das anschließende Abkühlen bis auf 80° C bedarf in etwa eines Zeitraums von 80 Minuten. Folglich ist mit der erfindungsgemäßen Vorrichtung (12) bzw. (100) pro Aufnahmebehälter (20), (22) ein Durchsatz von 15 Liter/Stunde möglich.

Der Untertisch (10) kann isoliert und mit einem Blechmantel verkleidet sein.

## Patentansprüche

1. Vorrichtung (12, 100) zum Sterilisieren von Abwasser wie infektiösem oder gentechnisch belastetem Abwasser umfassend zumindest einen ersten und einen zweiten Aufnahmebehälter (20, 22), wobei jeder Aufnahmebehälter der verfahrbar ausgebildeten Vorrichtung (12, 100) als Abwasser sammelnder kontrolliert kühlbarer Sterilisationsbehälter ausgebildet ist und jeweils sowohl eine Heizeinrichtung (42, 44, 114, 116) als auch ein Kühlelement einer Kühleinrichtung (50) aufweist.

2. Vorrichtung nach Anspruch 1,
**dadurch gekennzeichnet,**
daß die Heizeinrichtung ein im jeweiligen Aufnahmebehälter (20, 22) verlaufendes Heizrohr (114, 116) ist, wobei die Heizrohre vorzugsweise von einem einzigen Dampferzeuger (124) ausgehen, der über Steuerventile (118, 120, 124) mit den Heizrohren verbindbar ist.

3. Vorrichtung nach Anspruch 1 oder 2,
**dadurch gekennzeichnet,**
daß die Rückkühleinrichtung eine in der Vorrichtung (12, 100) angeordnete Kühlmaschine (50), insbesondere luftgekühlte Kältemaschine mit geschlossenem Kältekreislauf ist.

4. Vorrichtung nach zumindest einem der vorhergehenden Ansprüche,
**dadurch gekennzeichnet,**
daß die Vorrichtung (12, 100) als verfahrbarer Untertisch (10) ausgebildet ist, wobei der Untertisch insbesondere einen Wasserauslaß (130) wie -zapfhahn, der vorzugsweise über einen Näherungsschalter (132) betätigbar ist, und/oder eine obere gegebenenfalls Luftaustrittsöffnungen (60) einer Kältemaschine (50) aufweisende Abdeckung (28) aufweist, in der eine das Abwasser in einen der als vorzugsweise aus Edelstahl bestehenden Druckbehälter ausgebildeten Aufnahmebehälter (20, 22) leitende Zuführung wie Trichter (30) oder Becken (32) angeordnet ist.

5. Vorrichtung nach zumindest einem der vorhergehenden Ansprüche,
**dadurch gekennzeichnet,**
daß vorzugsweise über Magnetventile (76, 78) absperrbare Leitungen der Aufnahmebehälter (20, 22) in eine gemeinsame, ein sterilisiertes und rückgekühltes Abwasser förderndes Aggregat wie Zahnradpumpe (80) aufweisende Leitung (82) übergehen, die über eine Verbindung wie Schlauch (84) mit einem Entwässerungssystem verbindbar ist, wobei der Schlauch (84) vorzugsweise über eine an einer Seite (54) des Untertischs (10) angeordnete Schnellschlußkupplung (86) mit der das Förderaggregat (80) aufweisenden Leitung (82) verbunden ist.

6. Vorrichtung nach zumindest einem der vorhergehenden Ansprüche,
**dadurch gekennzeichnet,**
daß der Schlauch (84) oberhalb der Aufnahmebehälter (20, 22) in dem Entwässerungssystem mündet.

7. Vorrichtung nach zumindest einem der vorhergehenden Ansprüche,
**dadurch gekennzeichnet,**
daß in jedem Aufnahmebehälter (20, 22) ein Temperaturfühler (70, 72) und/oder ein Füllstandsmeßelement (38, 40) angeordnet ist.

8. Vorrichtung nach zumindest einem der vorhergehenden Ansprüche,
**dadurch gekennzeichnet,**
daß die Kühlelemente (46, 48) mit Kühlwasser wie einem Trinkwassemetz (102) entnommenem Leitungswasser durchströmbar sind.

9. Verfahren zum Sterilisieren von vorzugsweise hochgradig infektiösem und/oder gentechnisch belastetem Abwasser, wobei das Abwasser wahlweise einem von zwei in einem verfahrbaren Untertisch angeordneten Aufnahmebehältern (20, 22) zugeführt wird, das zu sterilisierendes Abwasser in einem der Behälter auf eine Temperatur T1 von zumindest 121° C erhitzt wird, daß nach erfolgter Sterilisation das Abwasser auf eine Temperatur T2 von zumindest 80° C abgekühlt und sodann einem Entwässerungssystem zugeführt wird, wobei während des Abkühlens der andere Aufnahmebehälter mit weiteren zu sterilisierenden Abwasser gefüllt und/oder in dem Behälter eingefülltes Abwasser sterilisiert wird.

10. Verfahren nach Anspruch 9,
**dadurch gekennzeichnet,**
daß zu sterilisierendes Abwasser vorzugweise über einen Zeitraum von zumindest 20 Minuten auf einer Temperatur T1 von 134° C gehalten wird und/oder das sterilisierte Abwasser auf eine Temperatur T2 von zumindest 80° C abgekühlt wird und anschließend einem üblichen Entwässerungssystem zugeführt wird.

## Claims

1. A device (12, 100) for sterilising waste water such as infectious waste water or waste water that has been contaminated by genetic engineering, comprising at least a first and a second receiving vessel (20, 22), wherein each receiving vessel of the movably designed device (12, 100) takes the form of a sterilisation vessel which collects waste water and which is capable of being cooled in controlled manner and each receiving vessel comprises both a heating arrangement (42, 44, 114, 116) and a cooling element pertaining to a cooling arrangement (50).

2. Device according to Claim 1,
characterised in that
the heating arrangement is a heating pipe (114, 116) extending within the respective receiving vessel (20, 22), said heating pipes preferably emanating from a single steam generator (124) which is capable of being connected to the heating pipes via control valves (118, 120, 124).

3. Device according to Claim 1 or 2,
characterised in that
the recooling arrangement is a refrigerating machine (50), in particular an air-cooled refrigerating machine with a closed refrigeration cycle, which is arranged in the device (12, 100).

4. Device according to at least one of the preceding claims,
characterised in that
the device (12, 100) takes the form of a movable undertable (10), said undertable comprising, in particular, a water outlet (130), such as a water tap which is preferably capable of being actuated via a proximity switch (132), and/or an upper cover (28), where appropriate comprising air-exit holes (60) pertaining to a refrigerating machine (50), in which a feed such as a funnel (30) or basin (32) is arranged which conducts the waste water into one of the receiving vessels (20, 22) which take the form of pressure vessels preferably consisting of high-grade steel.

5. Device according to at least one of the preceding claims,
characterised in that
lines pertaining to the receiving vessels (20, 22), which are capable of being shut off preferably via solenoid valves (76, 78), pass into a common line (82) comprising a unit conveying sterilised and recooled waste water, such as a gear pump (80), said common line being capable of being connected to a drainage system via a connection such as a hose (84), said hose (84) preferably being connected, via a quick-closing coupling (86) arranged on one side of the undertable (10), to the line (82) comprising the conveying unit (80).

6. Device according to at least one of the preceding claims,
characterised in that
the hose (84) discharges into the drainage system above the receiving vessels (20, 22).

7. Device according to at least one of the preceding claims,
characterised in that
a temperature sensor (70, 72) and/or a filling-level gauge (38, 40) is arranged in each receiving vessel (20, 22).

8. Device according to at least one of the preceding claims,
characterised in that
the cooling elements (46, 48) are capable of being flowed through by cooling water such as tap water which has been withdrawn from a drinking-water supply system (102).

9. A process for sterilising preferably highly infectious waste water and/or waste water that has been contaminated by genetic engineering, wherein the waste water is optionally supplied to one of two receiving vessels (20, 22) arranged in a movable undertable, the waste water to be sterilised is heated in one of the vessels to a temperature T1 of at least 121°C, in that after sterilisation has taken place the waste water is cooled to a temperature T2 of at least 80°C and is then supplied to a drainage system, whereby during the cooling the other receiving vessel is filled with further waste water to be sterilised and/or waste water which has been charged into the vessel is sterilised.

10. Process according to Claim 9,
characterised in that
waste water to be sterilised is preferably maintained at a temperature T1 of 134°C over a period of at least 20 minutes and/or the sterilised waste water is cooled to a temperature T2 of at least 80°C and is subsequently supplied to a conventional drainage system.

## Revendications

1. Dispositif (12, 100) pour stériliser des eaux résiduaires ainsi que des eaux infectées ou chargées de gênes, comprenant au moins un premier et deuxième récipient (20, 22), chaque récipient du dispositif mobile (12, 100) étant réalisé sous la forme d'un réservoir de stérilisation, refroidissable de manière contrôlée et collectant des eaux résiduaires, et ayant chacun à la fois une installation de chauffage (42, 44, 114, 116) et un élément refroidisseur d'une installation de refroidissement (50).

2. Dispositif selon la revendication 1,
caractérisé en ce que
l'installation de chauffage se compose de tubes chauffants (114, 116) passant dans chaque récipient (20, 22), les tubes chauffants partant de préférence d'un unique générateur de vapeur (124) relié par des vannes de commande (118, 120, 124) aux tubes chauffants.

3. Dispositif selon l'une quelconque des revendications 1 ou 2,
caractérisé en ce que
l'installation de refroidissement est une machine frigorifique (50) installée dans le dispositif (12, 100), notamment une machine frigorifique refroidie par de l'air et ayant un circuit frigorifique fermé.

4. Dispositif selon l'une quelconque des revendications précédentes,
caractérisé en ce que
le dispositif (12, 100) est sous la forme d'un meuble bas (10), mobile, et ce meuble bas comporte notamment une sortie d'eau (130) telle qu'un robinet, de préférence actionné par un commutateur de proximité (132), et/ou en haut un couvercle (28) ayant le cas échéant plusieurs orifices de sortie d'air (60) d'une machine frigorifique (50), ce couvercle étant muni d'une conduite telle qu'un entonnoir (30) ou une cuvette (32) conduisant l'eau résiduaire dans un des récipients (20, 22), de préférence en forme de réservoir sous pression en acier inoxydable.

5. Dispositif selon au moins l'une quelconque des revendications précédentes,
caractérisé en ce que
des conduites, fermées de préférence par des électrovannes (76, 78) des récipients (20, 22), passent dans une conduite (82) commune, équipée d'une unité de transfert des eaux résiduaires stérilisées et refroidies, telle qu'une pompe à engrenage (80), et pouvant être reliée par une liaison telle qu'un tuyau (84) à un système d'évacuation des eaux, le tuyau (84) étant relié de préférence par un accouplement rapide (86) prévu sur un côté (54) du meuble bas (10) avec la conduite (82) munie de l'unité de transfert (80).

6. Dispositif selon au moins l'une quelconque des revendications précédentes,
caractérisé en ce que
le tuyau (84) débouche dans le système d'évacuation des eaux, au-dessus des récipients (20, 22).

7. Dispositif selon au moins l'une quelconque des revendications précédentes,
caractérisé en ce que
chaque récipient (20, 22) comporte un capteur de température (70) et/ou un élément de mesure de niveau (38, 40).

8. Dispositif selon au moins l'une quelconque des revendications précédentes,
caractérisé en ce que
les éléments de refroidissement (46, 48) sont traversés par de l'eau de refroidissement provenant du réseau d'eau potable (102).

9. Procédé pour stériliser notamment des eaux résiduaires fortement infectées et/ou chargées de gênes,
dans lequel
des eaux résiduaires sont recueillies dans deux récipients (20, 22) prévus dans un meuble bas, mobile, les eaux résiduaires à stériliser sont chauffées dans l'un des récipients à une température T1 au moins égale à 121°C, puis après la stérilisation, elles sont refroidies à une température T2 d'au moins 80°C, et aussitôt évacuées dans un système d'évacuation d'eau, tandis que pendant le refroidissement on remplit l'autre récipient avec des eaux résiduaires à stériliser et/ou on stérilise les eaux résiduaires déjà introduites dans le récipient.

10. Procédé selon la revendication 9,
caractérisé en ce que
les eaux résiduaires à stériliser sont maintenues à une température T1 de 134°C pendant une durée d'au moins 20 minutes et/ou les eaux résiduaires stérilisées sont refroidies à une température T2 d'au moins 80°C, puis on les évacue dans un système d'évacuation usuel.
